# EUROPEAN PATENT APPLICATION

(11) **EP 3 228 306 A1**
(43) Date of publication of application: **11.10.2017**
(21) Application number: 16163764.0
(22) Date of filing: 04.04.2016
(51) Int. Cl.: A61K 9/00, A61K 47/69

(54) **COMPLEX COMPOUND COMPRISING OBETICHOLIC ACID AND CYCLODEXTRIN AND PHARMACEUTICAL FORMULATION COMPRISING THE COMPLEX COMPOUND**

(71) Applicant: ratiopharm GmbH, 89079 Ulm (DE)
(72) Inventor: ALBRECHT, Wolfgang, 89075 Ulm (DE); GEIER, Jens, 72534 Hayingen (DE); GUSERLE, Richard, 89259 Kötz (DE); MATHIEU, Alexandre, 89075 Ulm (DE); JEGOROV, Alexandr, 37316 Dobrá Voda (CZ)
(74) Representative: Ter Meer Steinmeister & Partner

(57) **Abstract**

The present invention relates to a complex compound comprising obeticholic acid and cyclodextrin and a pharmaceutical formulation comprising it. The invention further relates to a process for producing said pharmaceutical formulation. Finally, the invention relates to the therapeutic use of the complex compound in the treatment of a farnesoid X receptor (FXR) mediated disease or condition.

## Description

### Background of the invention

The present invention relates to a complex compound comprising obeticholic acid and cyclodextrin and a pharmaceutical formulation comprising it. The invention further relates to a process for producing said pharmaceutical formulation. Finally, the invention relates to the therapeutic use of the complex compound in the treatment of a farnesoid X receptor (FXR) mediated disease or condition.

Obeticholic acid (OCA, lab-code: INT-747) is a bile acid derivative and potent and selective agonist of the farnesoid X receptor (FXR). FXR controls bile acid homeostasis and its activation leads to an inhibition of the conversion of cholesterol into bile acid and prevents from the accumulation in the liver. Obeticholic acid is currently evaluated for the treatment of alcoholic hepatitis, of nonalcoholic steatohepatitis (NASH) and of primary biliary cirrhosis.

In the context of this invention, the term "obeticholic acid" is understood to mean 6α-ethyl-chenodeoxycholic acid (also: 3α,7α-dihydroxy-6α-ethyl-5β-cholan-24-oic acid) in accordance with the following chemical formula (1).

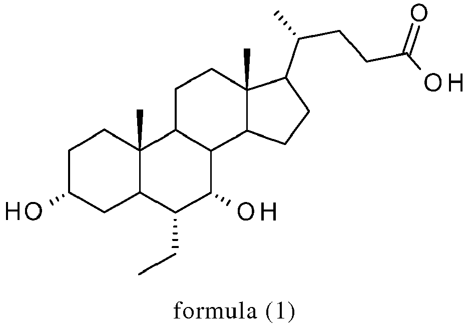

The compound can be present in the form of different stereoisomers. In the context of this invention, obeticholic acid is preferably present in the form of (4R)-4-[(3R,5S,6R,7R,8S,9S,10S,13R,14S,17R)-6-ethyl-3,7-dihydroxy-10,13-dimethyl-2,3,4,5,6,7,8,9,11,12,14,15,16,17-tetradecahydro-1H-cyclopenta[a]phenanthren-17-yl]pentanoic acid, as depicted in the above formula (1).

Synthesis pathways for amorphous obeticholic acid, a crystalline obeticholic acid Form C and the therapeutic use of obeticholic acid have been described in WO 2013/192097. Salts, solvates and amino acid conjugates of obeticholic acid are further described in WO 2002/072598.

The thermodynamic pKₐ of obeticholic acid is about 5 and when protonated, e.g. in gastric fluid with pH about 2, obeticholic acid is poorly soluble in water. At pH above 7 the ionized and soluble species of obeticholic acid is being formed.

Obeticholic acid is a bile acid derivative with pH-dependent aqueous solubility. This pH-dependency is particularly relevant in the physiological pH-range, i.e. between pH about 2 (gastric fluid) and small intestine (pH 4-7). Based on the large inter-individual variability of the pH in the fluids in the gastro-intestinal lumen and its impact on the solubility of obeticholic acid, after oral administration of a therapeutic dose, a considerable inter-subject variability of drug absorption and bioavailability is expected.

A recent analysis of the duodenal fluid in the fasted and fed state in particular demonstrated that in the fasted state highly variable pH values were observed, ranging from 3.4 to 8.3. In such a wide pH-range, obeticholic acid would remain either undissolved (pH <5) or dissolved (pH >7). As the absorption from the intestinal tract usually correlates with dissolution and solubility, a high inter-individual variability of drug absorption would be expected.

Due to the variable solubility at different pH, likewise the bioavailability of obeticholic acid depends on the pH of the surrounding body fluid, such as in gastric fluid. Thus, it is difficult to provide obeticholic acid in a pharmaceutically acceptable form from which the drug is released with constant rate.

### Technical problem

Hence, there was a need in the art, which overcomes the drawbacks of the prior art formulations. In particular, there was a need in the art to provide obeticholic acid in a form, which has pH-independent solubility, in particular in body fluid, such as gastric fluid, i.e. a pH-independent solubility within a pH range from 2 to 7.

In particular, it was an object of the present invention to provide obeticholic acid in a form in which it can be processed into a dosage form, preferably a dosage form suitable for oral administration, which (even after storage) releases the active agent obeticholic acid at constant rate.

Further, obeticholic acid should be provided in a form, which is stable even during long term-storage and even at different storage conditions, such as different temperature and/or different humidity. Preferably, storage stability for 12 months at 40°C and 75% humidity should be achieved.

Finally, the intention is also to provide dosage forms of obeticholic acid which ensure high and pH-independent solubility and bioavailability at the same time.

All the above-mentioned objectives are supposed to be solved both for a formulation designed for immediate release (or "IR" for short) and for modified release (or "MR" for short).

### Brief description of Figures

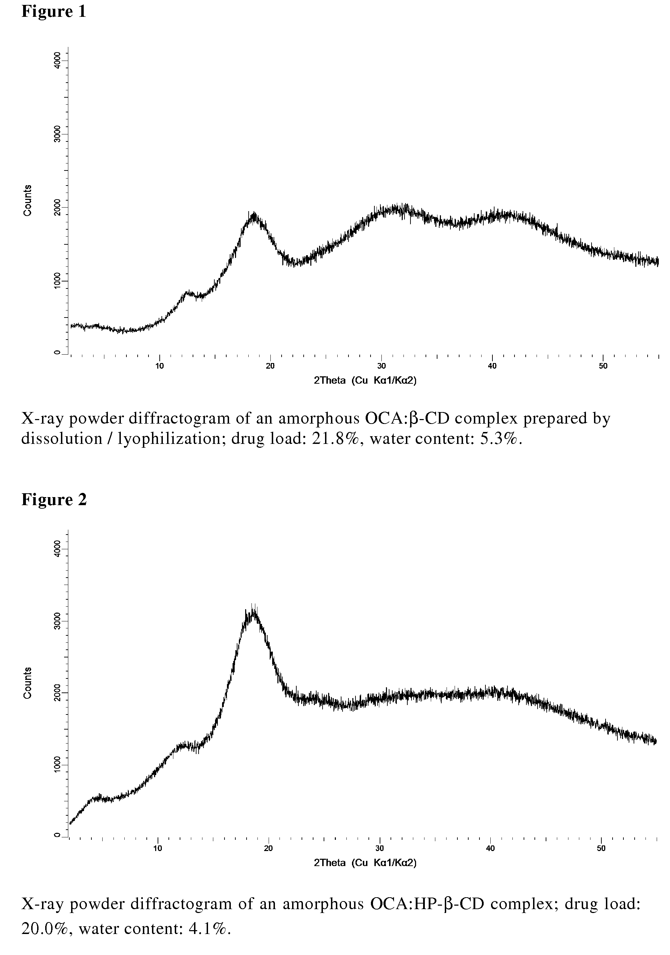

Figure 1: X-ray powder diffractogram (XRPD) of an amorphous obeticholic acid (OCA):β-Cyclodextrin (CD) complex prepared by dissolution/lyophilization.
Figure 2: XRPD of an amorphous OCA:Hydroxypropyl (HP)-β-CD complex

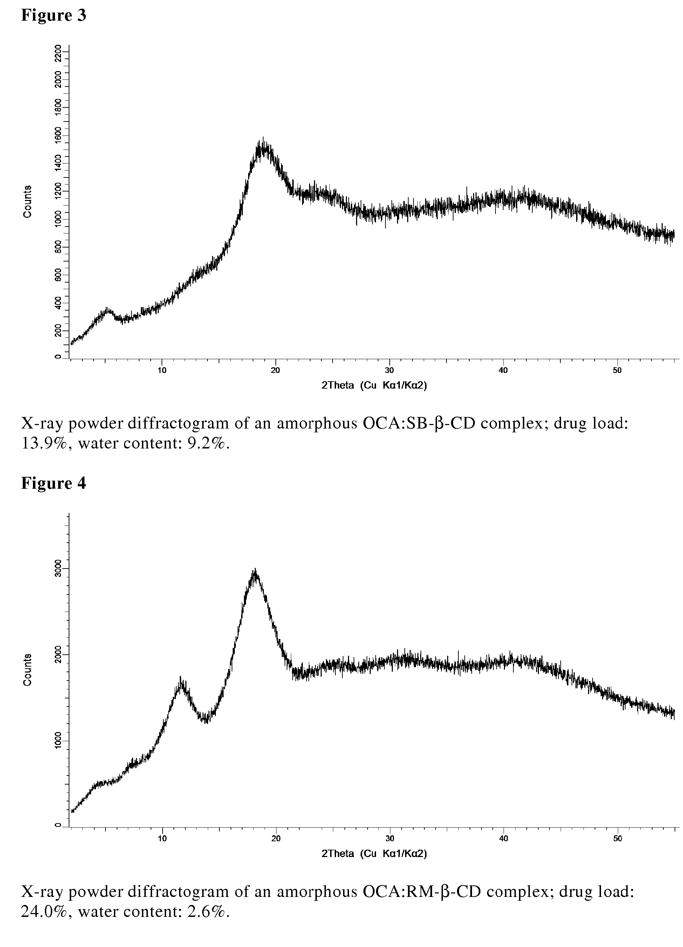

Figure 3: XRPD of an amorphous OCA:Sodium Sulfobutylether (SB)-β-CD complex.
Figure 4: XRPD of an amorphous OCA:Random methyl (RM)-β-CD complex.

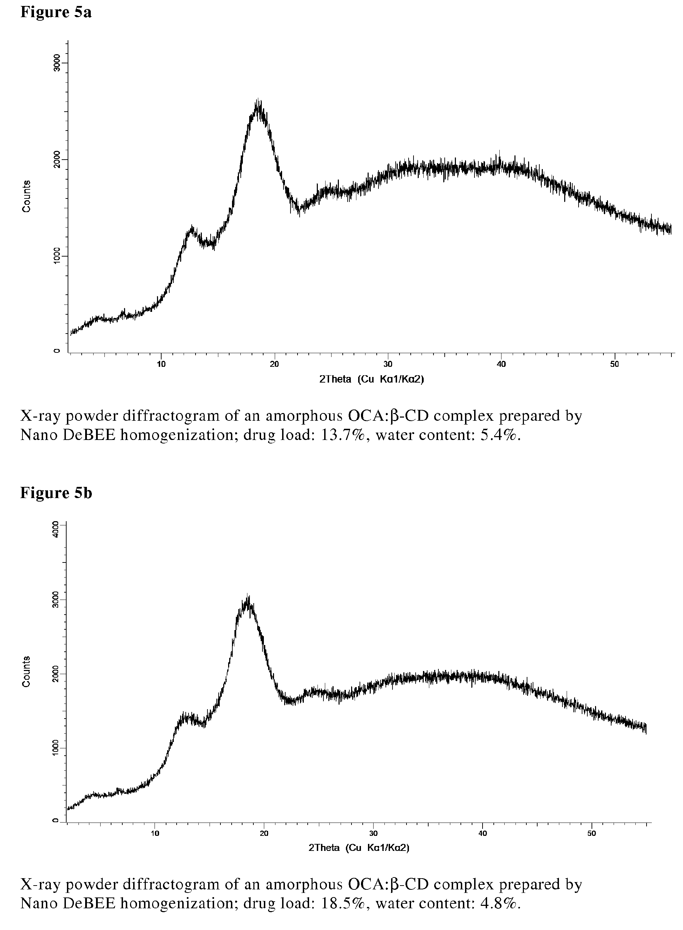

Figures 5a, b: XRPD of amorphous OCA:β-CD complexes prepared by Nano DeBEE high pressure homogenization.
Figure 6: XRPD of form W of OCA:β-CD complex.
Figure 7: XRPD of form H33 of OCA:β-CD complex.
Figure 8: Asymmetric unit of crystalline OCA:β-CD complex.
Figure 9: View along the a axis of the crystal structure of OCA:β-CD complex.
Figure 10: ¹³C CPMAS NMR spectra of crystalline OCA:β-CD complex, a physical mixture of amorphous OCA and β-CD, β-CD, and amorphous OCA.
Figure 11: Dynamic vapor sorption measurement of form H33 of OCA: β-CD complex.
Figure 12: Illustration of the relationship between dissolved OCA and CD-concentration.

### Summary of the invention

It has surprisingly been found in the present invention that the above objectives can be achieved by providing a complex compound comprising obeticholic acid and cyclodextrin. In the complex compound of the present invention obeticholic acid preferably is present in form of an inclusion complex and can be advantageously processed into pharmaceutical formulations.

Thus, the subject of the invention is a complex compound comprising (a) obeticholic acid and (b) cyclodextrin.

A further subject of the present invention is a pharmaceutical formulation, preferably in the form of an oral dosage form, in particular a solid dosage form, such as a capsule or tablet, comprising the complex compound of the present invention and optionally one or more pharmaceutical excipient(s).

A further subject of the present invention is a crystalline complex compound comprising:
(a) obeticholic acid and
(b) cyclodextrin,
wherein the complex is prepared by the steps of:
(i) concentrating or cooling and optionally adding seed crystals to a saturated solution of obeticholic acid and cyclodextrin in water,
(ii) isolating crystals of the complex compound from the suspension obtained in step (i), and
(iii) drying of the crystals in a desiccator over an aqueous solution of magnesium chloride containing excess solid magnesium chloride hexahydrate.

A further subject of the present invention is an amorphous complex comprising
(a) obeticholic acid and
(b) β-cyclodextrin,
wherein the complex is prepared by the steps of:
(i) suspending obeticholic acid and β-cyclodextrin in an aqueous solvent,
(ii) circulating the suspension in a high pressure homogenizer and
(iii) lyophilisation of the resulting suspension.

A further subject of the present invention is the use of the complex compound or the pharmaceutical formulation in medical treatment such as in the prevention or treatment of hepatic or biliary diseases and conditions.

The above-illustrated subjects of the present invention are alternative solutions to the above-outlined problems.

### Detailed Description of the Invention

### Complex compound

The term "obeticholic acid" (OCA) as used in the present application can refer to obeticholic acid according to the above formula (1), i.e. in the form of the free acid. Alternatively, it can refer to pharmaceutically acceptable salts, solvates, hydrates, polymorphs and mixtures thereof. Within the present application, ratios or amounts of obeticholic acid generally refer to the ratio or amount of obeticholic acid in form of the free acid.

In a particularly preferred embodiment the complex compound of the present invention as well as the pharmaceutical formulation of the present invention comprise obeticholic acid as the sole pharmaceutical active agent. In an alternative embodiment the complex compound of the present invention as well as the pharmaceutical formulation of the present invention can comprise obeticholic acid in combination with further pharmaceutical active agent(s).

In line with the present application the term "cyclodextrin" may refer to nonreducing cyclic saccharides and mixtures thereof. Preferably, said cyclic saccharides comprise six, seven, eight or nine glucose units, linked by alpha-1,4 interglycosidic bonds.

Cyclodextrin (CD) can be a naturally occurring cyclodextrin or a chemically modified cyclodextrin. The cyclodextrins of the present invention can be (partially) substituted. Substitution can be achieved with acetyl groups, alkoxy groups such as carboxymethyl, heteroaromatic or aromatic groups such as benzyl, heteroalkyl or alkyl groups, preferably C₁-C₈ alkyl groups such as methyl, ethyl, propyl, butyl and pentyl, or with hydroxyalkyl groups such as hydroxyethyl and hydroxypropyl. Preferably, the cyclodextrins can be (partially) substituted with hydroxypropyl groups.

The average degree of substitution is usually 0.1 to 3, preferably 0.3 to 2, more preferably 0.4 to 1.5 and most preferably 0.5 to 1 per glucose unit. In case of (2-hydroxy)propyl-β-cyclodextrin, the degree of substitution is 0.1 to 2, preferably 0.3 to 1.5 and most preferably 0.5 to 1 of hydroxypropyl groups per glucose unit.

Examples of cyclodextrins are α-cyclodextrin (α-CD), β-cyclodextrin (β-CD), 2-hydroxypropyl-β-cyclodextrin (HP-β-CD), randomly methylated β-cyclodextrin, (RM-β-CD), sodium sulfobutylether-β-cyclodextrin (SB-β-CD), γ-cyclodextrin (y-CD) and 2-hydroxypropyl-γ-cyclodextrin (HP-γ-CD). Preferred are β-cyclodextrin, and 2-hydroxypropyl-β-cyclodextrin (HP-βCD), most preferably β-cyclodextrin.

Furthermore, it is particularly preferred that in all embodiments of the present invention cyclodextrins can be used in the form of cyclodextrin hydrate. In a preferred embodiment the water content of the cyclodextrin used for making the complex can be 10 to 15 wt.% based on the total weight of the cyclodextrin.

In a preferred embodiment, obeticholic acid (a) and cyclodextrin (b) are present in the form of an inclusion complex. The complex, preferably the inclusion complex, can preferably lead to a solid form of obeticholic acid, preferably to a form of molecular dispersity. The solid form of obeticholic acid can be regarded as obeticholic acid in form of a solid solution of obeticholic acid. This novel solid form of obeticholic acid is preferably a stabilized and/or hydrophilized form of the active agent.

In the complex compound of the present invention, the obeticholic acid molecule is typically included in the molecular cavities of the cyclodextrin molecule, i.e. obeticholic acid is only present within cyclodextrin molecules. Thus, typically no adsorbed, un-entrapped obeticholic acid occurs.

Typically, in the inclusion complex of the present invention obeticholic acid and cyclodextrin are non-covalently linked, i.e. the complex can be described as a non-covalent inclusion complex. Obeticholic acid and cyclodextrin may be linked in the complex compound by one or more hydrogen bonds.

Furthermore, all preferred inclusion complexes of the present invention can be supramolecular inclusion complexes. In particular, all preferred inclusion complexes of the present invention can be non-covalent and supramolecular inclusion complexes. The term "supramolecular" is understood as describing self-organizing molecular interactions that result in the formation of new structures that stay together without establishing a covalent linkage.

The complex compound of the present invention can preferably be present in a solid state form, such as a crystalline form or an amorphous form, wherein the crystalline form is preferred.

A solid state form may be referred to herein as being characterized by data selected from two or more different data groupings, for example, by a powder XRD pattern having a group of specific peaks; or by a powder XRD pattern as shown in a figure depicting a diffractogram, or by "a combination thereof (or "combinations thereof" or "any combination thereof"). These expressions, e.g., "any combination thereof" contemplate that the skilled person may characterize for example a crystal form using any combination of the recited characteristic analytical data. For example, the skilled person may characterize a crystal form using a group of three, four or five characteristic powder XRD peaks, and supplement that characterization with one or more additional features observed in the powder X-ray diffractogram, e.g., an additional peak, a characteristic peak shape, a peak intensity, or even the absence of a peak at some position in the powder XRD pattern. Alternatively, the skilled person may in some instances characterize a crystal form using a group of three, four or five characteristic powder XRD peaks and supplement that characterization with one or more additional features observed using another analytical method, for example, using one or more characteristic peaks in a solid state IR spectrum, or characteristics of the DSC thermogram of the crystal form that is being characterized.

Unless indicated otherwise, XRPD peaks are recorded using copper Kα₁/ Kα₂ radiation with wavelength 1.5419 Å (weighted mean of Cu Kα₁ and Cu Kα₂). Further, unless indicated otherwise, XRPD peaks are reported as degrees 2-theta values with standard errors of ± 0.2 degrees 2-theta.

A solid state form may be referred to herein as being characterized by graphical data "as depicted in" a particular figure. Such data include, for example, powder X-ray diffractograms. For example an amorphous solid state is characterized by a halo-like picture of the resulting powder X-ray diffractogram. The skilled person will understand that such graphical representations of data may be subject to small variations, e.g., in peak relative intensities and peak positions due to factors such as variations in instrument response and variations in sample preparation, which are well known to the skilled person. Nonetheless, the skilled person would readily be capable of comparing the graphical data in the figures herein with graphical data generated for an unknown solid state form and for example for a crystal form, confirm whether the two sets of graphical data are characterizing the same crystal form or two different crystal forms.

Changes in the position and intensity of the XRPD peaks of the crystalline complex compounds of the present invention may occur dependent on the relative humidity of the atmosphere surrounding the crystals and/or the water content of the crystals.

The obeticholic acid : cyclodextrin (OCA:CD) complexes may be prepared by crystallisation or lyophilization of an aqueous solution containing obeticholic acid and cyclodextrin, as described in the present application.

In one embodiment of the present invention the crystalline complex of obeticholic acid and beta-cyclodextrin (molar ratio about 1:1) at about 33% relative humidity (also designated in the following as Form H33) is characterized by the following XRPD diffraction peaks: 4.2; 5.5; 6.0; 6.6; 6.7; 7.6; 7.9, 8.3; 9.1; 9.6; 10.2; 10.6; 10.8; 10.9; 11.2; 11.7; 12.4; 12.9; 13.3; 14.1; 14.8; 15.2; 15.4; 15.9; 16.2; 16.4; 16.8; 17.3; 17.7; 18.0; 18.2; 18.6; 18.9; 19.2; 19.5; 19.6; 19.8 and 20.1 degrees 2-theta ± 0.2 degrees 2-theta. "33% Relative humidity" refers to the equilibrium humidity over a saturated solution of magnesium chloride in water, containing excess solid magnesium chloride hexahydrate, at a temperature of 23°C (cf. L. Greenspan, Journal of Research of the National Bureau of Standards 1977, 81A, 89; A. Wexler, S. Hasegawa, Journal of Research of the Natiortal Bureau of Standards 1954, 53, 19).

In a preferred embodiment of the present invention the crystalline complex of obeticholic acid and beta-cyclodextrin at about 33% relative humidity is characterized by the following XRPD diffraction peaks: 5.5; 7.6; 10.9; 12.4; and 18.2 degrees 2-theta ± 0.2 degrees 2-theta.

In a further preferred embodiment of the present invention the crystalline complex of obeticholic acid and beta-cyclodextrin at about 33% relative humidity is further characterized by the following XRPD diffraction peaks: 9.6; 10.8; 14.1; 15.9; 16.4; and 18.0 degrees 2-theta ± 0.2 degrees 2-theta.

In an alternative preferred embodiment the complex, more preferably the inclusion complex, comprising obeticholic acid and cyclodextrin can preferably be present in a non-crystalline, preferably in amorphous form.

The complex compound of the present invention can preferably have a molecular ratio of obeticholic acid (a) to cyclodextrin (b) from 5:1 to 1:10, preferably from 3:1 to 1:8, more preferably from 2:1 to 1:5, most preferably from 1:1 to 1:3.

Generally, the complex compound of the present invention comprising a) obeticholic acid and b) cyclodextrin can be achieved if the molecular ratio of obeticholic acid (a) to cyclodextrin (b) is as mentioned above. In a further preferred embodiment obeticholic acid (a) and the cyclodextrin (b) may form a complex, preferably an inclusion complex, in which the molar ratio of obeticholic acid to cyclodextrin is from 0.5:1 to 2:1, preferably from 0.7:1 to 1.5:1, more preferably from 0.8:1 to 1.2:1, especially about 1:1.

### Process of producing the complex compound

The present invention further relates to processes for producing the complex compound comprising obeticholic acid and cyclodextrin, preferably as inclusion complex. Generally, the comments made above for the complex compound comprising obeticholic acid and cyclodextrin may also apply to the processes of the present invention.

Hence, a further subject of the present invention is a process for producing a complex compound comprising obeticholic acid and cyclodextrin, the process comprising the steps of
i) dissolving and/or dispersing obeticholic acid and cyclodextrin in a solvent;
ii) optionally homogenizing the solution or dispersion from step (a); and
iii) removing the solvent and optionally isolating the composition.

In a preferred embodiment, the present invention refers to a process for preparing a crystalline complex compound as described above, the process comprising the steps of:
i) concentrating or cooling a saturated solution of obeticholic acid and cyclodextrin in a solvent,
ii) isolating crystals of the complex compound from the suspension obtained in step i), and
iii) drying of the crystals in a desiccator over an aqueous solution of magnesium chloride containing excess solid magnesium chloride hexahydrate.

In this embodiment, step i) may optionally include the addition of seed crystals to the saturated solution.

In another preferred embodiment, the present invention refers to a process for preparing an amorphous complex compound as described above, the process comprising the steps of:
i) suspending obeticholic acid and cyclodextrin in a solvent,
ii) circulating the suspension in a high pressure homogenizer and
iii) lyophilisation of the resulting suspension.

In this embodiment, the cyclodextrin is preferably β-cyclodextrin.

Further, step ii) is typically performed for 5 to 240 min, preferably from 10 to 120 min, more preferably from 20 to 90 min, most preferably from 30 to 60 min.

In this embodiment, step ii) is further typically performed with a pressure of 2,500 to 20,000 psi, preferably from 5,000 to 20,000 psi, most preferably from 7,500 to 15,000 psi.

In the above processes, the solvent used in step i) is preferably an aqueous solvent, most preferably water.

### Pharmaceutical formulations

A further subject of the present invention is a pharmaceutical formulation, preferably in form of a solid oral dosage form, comprising the complex compound according to the present invention and optionally one or more pharmaceutical excipient(s).

The one or more excipients(s) (c) may be selected from surfactants (c1), wicking agents (c2), fillers (c3), binders (c4), disintegrants (c5), lubricants (c6), glidants (c7) and plasticizers (c8). Suitable excipients are for example disclosed in "Lexikon der Hilfsstoffe für Pharmazie, Kosmetik und angrenzende Gebiete", published by H.P. Fielder, 4th Edition and "Handbook of Pharmaceutical Excipients", 3rd Edition, published by A.H. Kibbe, American Pharmaceutical Association, Washington, USA, and Pharmaceutical Press, London.

Surfactants (c1) can be regarded as substances lowering the interfacial tension between two phases, thus enabling or supporting the formation of dispersions or working as a solubilizer. Common surfactants are alkylsulfates (for example sodium lauryl sulfate), alkyltrimethylammonium salts, alcohol ethoxylates and the like. Surfactants can be used in an amount of 0.05 to 2% by weight, preferably of 0.1 to 1.5% by weight, based on the total weight of the pharmaceutical formulation.

Wicking agents (c2) can be regarded as substances with the ability to draw a biological fluid (preferably water) into a solid, preferably by physisorption. Physisorption is defined as a form of adsorption in which the solvent molecules can loosely adhere to the surfaces of the wicking agent, preferably via van der Waals interaction between the surface of the wicking agent and the adsorbed fluid molecule (preferably water). Usually, a wicking agent can do this with or without swelling. For example, microcrystalline cellulose can be used as wicking agent. Wicking agents (c2) can be used in an amount of 0 to 40% by weight, preferably 1 to 15% by weight, based on the total weight of the pharmaceutical formulation.

Fillers (c3) or diluents can be used to increase the bulk volume and weight of a low-dose drug to a limit at which a pharmaceutical dosage from can be formed. Fillers should fulfil several requirements, such as being chemically inert, non-hygroscopic, biocompatible, easily processible and possessing good biopharmaceutical properties. Examples of fillers are lactose, sucrose, glucose, mannitol, calcium carbonate, cellulose and others. Fillers (c3) can be used in an amount of 0 to 60% by weight, preferably 1 to 20% by weight, based on the total weight of the pharmaceutical formulation.

Binders (c4) may be added to the pharmaceutical formulation in order to ensure that oral dosage forms, preferably tablets, can be formed with the required mechanical strength. The binder can, for example, be starch, polyvinyl pyrrolidone or cellulose derivates. The binding agent can be present in an amount of 0 to 80% by weight, based on the total weight of the pharmaceutical formulation.

Disintegrants (c5) are compounds, which enhance the ability of the dosage form, preferably the ability of the tablet when in contact with a liquid, preferably water, to break into smaller fragments. Preferred disintegrants are sodium carboxymethyl starch, cross-linked polyvinyl pyrrolidone (crospovidone), sodium carboxymethyl glycolate (for example Explotab^{®}), swelling polysaccharide, for example soy polysaccharide, carrageenan, agar, pectin, starch and derivates thereof, protein, for example formaldehyde-casein, sodium bicarbonate or mixtures thereof. Disintegrants can be used in an amount of 0 to 20% by weight, preferably of 1 to 10% by weight, based on the total weight of the pharmaceutical formulation.

The function of lubricants (c6) is reported to ensure that tablet formation and ejection can occur with low friction between the solid and the die wall. The lubricant is preferably a stearate or fatty acid, more preferably an earth alkali metal stearate, such as magnesium stearate. The lubricant is suitably present in an amount of 0 to 2% by weight, preferably of about 0.1 to 1.0% by weight, based on the total weight of the pharmaceutical formulation. Lubricants can generally increase the powder flowability.

Glidants (c7) can also be used to improve the flowability. Traditionally, talc was used as glidant, but is nowadays nearly fully replaced by colloidal silica (for example Aerosil^{®}). Preferably, the glidant agent is present in an amount of up to 3% by weight, based on the total weight of the pharmaceutical formulation.

Plasticizers (c8) usually are reported to be compounds capable of lowering the glass transition temperature (Tg) of a non-erodible material, preferably of lowering T_{g} from 1 to 50°C. Plasticizers (c8) usually are low molecular weight compounds (having a molecular weight of 50 to 500 g/mol) and can comprise at least one hydrophilic group. Examples of suitable plasticizers are dibutyl sebacetate (DBS), Myvacet^{®} (acetylated monoglycerides), triacetin (GTA), citric acid esters, like acetyltriethyl citrate (ATEC) or triethyl citrate (TEC), propylene glycol, dibutyl phthalate, diethyl phthalate, or mixtures thereof.

It lies in the nature of pharmaceutical recipients that they sometimes can perform more than one function in a pharmaceutical formulation. In this regard it is generally noted that due to the nature of pharmaceutical recipients it cannot be excluded that a certain compound meets the requirements of more than one of the components (b) and (c1) to (c8). Therefore, cyclodextrin (b) may function as a component for forming the composition according to the invention as well as a pharmaceutical excipient (c), i.e. the fact that cyclodextrin is used as component for forming the composition according to the invention does not mean that it cannot also be acting as filler (c3).

However, in order to enable an unambiguous distinction, it is preferred in the present invention that one and the same pharmaceutical compound can only function as one of the compounds (b) or (c1) to (c8). For example, or if microcrystalline cellulose functions as a wicking agent (c2), it cannot additionally function as a disintegrant (c5), even though microcrystalline cellulose also exhibits a certain disintegrating effect.

The pharmaceutical formulation of the present invention can preferably comprise the following amounts of components:
10 to 1000 mg, preferably 20 to 500 mg, particularly 50 to 300 mg of the complex compound of the present invention,
0 to 1000 mg, preferably 15 to 500 mg, particularly 25 to 300 mg binder,
0 to 25 mg, preferably 1 to 15 mg, particularly 2 to 7 mg glidant,
0 to 20 mg, preferably 2 to 15 mg, particularly 4 to 10 mg surfactant,
0 to 15 mg, preferably 1 to 10 mg, particularly 2 to 8 mg lubricant, and
0 to 125 mg, preferably 5 to 100 mg, particularly 10 to 75 mg disintegrant.

In a preferred embodiment the pharmaceutical formulation of the present invention can preferably comprise:
1 to 80 wt.%, preferably 2 to 50 wt.%, particularly 5 to 30 wt.% of the complex compound of the present invention,
0 to 80 wt.%, preferably 2 to 50 wt.%, particularly 4 to 30 wt.% binder,
0 to 5 wt.%, preferably 0.1 to 1.5 wt.%, particularly 0.3 to 1.0 wt.% glidant,
0 to 2 wt.%, preferably 0.2 to 1.6 wt.%, particularly 0.4 to 1.4 wt.% surfactant,
0 to 2 wt.%, preferably 0.1 to 1.3 wt.%, particularly 0.2 to 0.8 wt.% lubricant,
0 to 20 wt.%, preferably 1 to 15 wt.%, particularly 2 to 10 wt.% disintegrant, based on the total weight of the pharmaceutical formulation.

The pharmaceutical formulation can be a solid oral dosage form, preferably a capsule or a tablet, more preferably a tablet for peroral use. Alternatively, the solid oral dosage form can be filled as powder or granulate into devices like sachets or stick-packs. Alternatively, the solid oral dosage form may be a capsule including an aqueous suspension of the complex compound of the present invention. Further, the capsule may include the complex compound of the present invention as wet crystals, i.e. the atmosphere surrounding the complex compound of the present invention, preferably in crystalline form, has a relative humidity of about 100%.

The oral dosage form, preferably a tablet or a capsule, may further be coated, preferably film coated.Typical film coatings of the dosage forms of the present invention include:
- film coating without affecting the release of the active ingredient,
- gastric juice-resistant film coatings,
- retard film coatings.

Generally, film coatings can be prepared by using film-forming agents such as waxes, cellulose derivatives, poly(meth)acrylate, polyvinylpyrrolidone, polyvinyl acetate phthalate, and/or shellac or natural rubbers such as carrageenan.

The gastric juice-resistant film coating preferably is a film coating being stable in the pH range of about 0.7 to 3.0, which is supposed to be the pH-value of human gastric juice found in the stomach. However, in an environment with a pH value of 5 to 9, which is supposed to be present in the (small) intestine of the human body, the gastric juice-resistant film coating preferably dissolves and the drug can be released.

The gastric juice-resistant film coating (often also referred to as enteric coating) can comprise film-forming agents, for example fats, fatty acids, waxes, alginates, shellac, polyvinyl acetate phthalate, cellulose derivatives such as carboxy methyl ethyl cellulose, cellulose acetate succinate, cellulose acetate phthalate, hydroxypropyl methyl cellulose phthalate, hydroxypropyl methyl cellulose acetate succinate, cellulose acetate trimellitate, and meth(acrylic)acid copolymers such as methyl acrylate-methacrylic acid copolymers, methyl methacrylate-methacrylic acid copolymers, Eudragits (for example Eudragit^{®} L30D, Eudragit^{®} L, Eudragit^{®} S).

The coating is preferably free of active ingredient. The thickness of the coating is usually 10 µm to 2 mm, preferably from 50 to 500 µm.

The preferred coating may comprise a film-forming agent and one or more of a lubricant, surfactant, glidant, pigment and water.

The preferred coating of the dosage form of the present invention can comprise, along with the film-forming agent, e.g. stearic acid as lubricant for plasticizing and dissolving the polymer, sodium lauryl sulfate as a surfactant for wetting and dispersing, talc as glidant, iron oxide yellow and/or titanium oxide as pigment(s) and optionally purified water.

### Process for producing the pharmaceutical formulation

Further, the subject of the present invention relates to a process for preparing a pharmaceutical formulation comprising the steps:
I) providing the complex compound according to the invention,
II) optionally adding further excipient(s) (c),
III) processing the mixture of step I) or II) to a solid oral dosage form, and
IV) optionally film-coating the oral dosage form.

In step I) a complex compound according to the present invention is provided, i.e. all the above process steps i), ii) and iii) leading to the present complex compound also apply to the process for preparing the pharmaceutical formulation.

In a preferred embodiment the solution or dispersion of the above-mentioned step ii) may be granulated before the solvent is removed in step iii). The resulting granules may further be processed in step II).

In step II) one or more further excipient(s) (c) can optionally be added to the complex compound of step I) or vice versa. During or after the addition of the optional excipients the mixture can preferably be blended and the resulting mixture may be preferably subjected to a granulation, such as a wet or a dry granulation.

In step III) one or more further excipient(s), such as lubricant, may be added and the mixture is processed into a solid oral dosage form. Processing the mixture into a solid oral dosage form can preferably comprise manufacturing the pharmaceutical formulation into tablets or filling the formulation into capsules.

### Medical use

The present invention further relates to the medical use of the complex compound of the present invention or the pharmaceutical formulation comprising it. In particular, the complex compound or the pharmaceutical formulation comprising it may be used in the treatment or prevention of an FXR mediated disease or condition, such as a hepatic or biliary disease or condition, in a subject in need thereof, in particular in a human patient.

The prevention or treatment may comprise administering an effective amount of the complex compound or the pharmaceutical formulation comprising it to the subject.

The disease or condition may be selected from biliary atresia, cholestatic liver disease, chronic liver disease, nonalcoholic steatohepatitis (NASH), hepatitis C infection, alcoholic liver disease, primary biliary cirrhosis (PBC), liver damage due to progressive fibrosis, liver fibrosis, and cardiovascular diseases including atherosclerosis, arteriosclerosis, hypercholesteremia, and hyperlipidemia. Most preferably, the disease or condition is alcoholic hepatitis, nonalcoholic steatohepatitis or primary biliary cirrhosis.

In a preferred embodiment the pharmaceutical composition can be administered one to three times a day, preferably once or twice a day, more preferably once a day.

The present invention is illustrated by the following examples.

### Experimental Part

All percentages given in the following refer to weight percent (wt.%), if not indicated otherwise.

Room temperature (RT) refers to a temperature of 23-25 °C, if not indicated otherwise.

### Analytical Methods

### X-ray Powder Diffraction (XRPD)

The samples were measured on a D8 Advance powder X-ray diffractometer (Bruker AXS, Karlsruhe, Germany) in a rotating PMMA sample holder (diameter: 25 mm; depth: 1 mm) in reflection mode (Bragg-Brentano geometry). The samples were covered with Kapton^{®} foil if protection against the atmosphere was necessary. Conditions of the measurements are summarized in the Table below. Raw data were analyzed with the program EVA (Bruker AXS, Karlsruhe, Germany). Background subtraction and Kα₂ stripping were not performed for the depicted diffractograms. Peak intensities were determined after background subtraction.

Conditions for powder diffraction measurements were as follows:

| | |
|---|---|
| radiation | Cu Kα₁/α₂ |
| source | 34 kV / 40 mA |
| detector | Vantec-1 (electronic window: 3°) |
| Kβ filter | Ni (diffracted beam) |
| measuring circle diameter | 435 mm |
| detector window slit | 12 mm |
| anti-scatter slit (diffracted beam) | 8 mm |
| divergence slit | v6.00 (variable) |
| Soller slit (incident /diffracted beam) | 2.5° |
| 2θ range | 2° ≤ 2θ ≤ 55° |
| step size | 0.016 |
| step time | 0.2 s |

### UHPLC/UV

Measurements were performed by using the following specifications:

| | |
|---|---|
| Instrument: | Agilent 1290 Infinity |
| Wavelength: | 200 nm (60 mm UV cell) |
| Column: | Acquity UPLC BEH -C18 1.7 µm 50 x 2.1mm |
| Column temp.: | 40.0 °C |
| Flow [mL/min]: | 0.5 |
| Injection volume: | 1 µL |
| Solvent A: | Water, pH 2.5 (adjusted with phosphoric acid) |
| Solvent B: | Acetonitrile |
| Gradient | Isocratic A/B (40/60 v/v) |

### Dynamic Vapor Sorption - Hygroscopy

Vapor sorption experiments were performed in the instrument SPSx-1µ (Projekt Messtechnik, Ulm, Germany) at 25°C and the humidity cycles as shown below.

| Cycle no. | rel. humidity (% RH) | | Number of | Time (h) |
|---|---|---|---|---|
| | start value | end value | steps | |
| 1 | 40 | 70 | 3 | |
| 2 | 70 | 75 | 1 | |
| 3 | 75 | 75 | 0 | 24 h* |
| 4 | 75 | 95 | 2 | |
| 5 | 95 | 90 | 1 | |
| 6 | 90 | 0 | 9 | |
| 7 | 0 | 5 | 1 | |
| 8 | 5 | 35 | 3 | |

| | | | | |
|---|---|---|---|---|
| * To investigate the absorption of water at the upper humidity level during stress conditions | | | | |

### Solubility Measurements

Obeticholic acid (OCA) or OCA: Cyclodextrin (CD) complex was weighed into a glass vial, followed by addition of 1 or 2 ml solvent (water, 0.01 N HCl, phosphate buffer pH 6.8 and FaSSIF). A stirring bar was added, the vial was fixed in a block heater at 37°C and the suspension was stirred with approx. 250 rpm. After 15 min and 1 h, samples were withdrawn, filtered through a 0.2 µm disposable filter, 100 µl of the clear filtrate were diluted with 900 µl acetonitrile (ACN):Water (1:1) and 1 µl thereof were analyzed by UHPLC/UV.

### Single-Crystal Diffraction

Single crystal diffraction data were collected on a Bruker D8 VENTURE system equipped with a multilayer monochromator and a Cu Kα Incoatec microfocus sealed tube (λ = 1.54178 Å) using combined ϕ and ω scans at 180 K.

### Solid-State ¹³C-CPMAS NMR Spectroscopy

¹³C CPMAS NMR spectra were measured on a Bruker Avance-III 400 MHz spectrometer (MAS rotation frequency 14 kHz; contact time 2 ms; recycle delay 5 s; acquisition time 75 ms; ¹H decoupling during acquisition with SPINAL-64 sequence).

### Differential Scanning Calorimetry (DSC)

The samples were placed in sealed aluminum crucibles (40 µL) with perforated lids (one hole in the centre, made by puncturing with a cannula of 0.6 mm diameter). The measured DSC curves are displayed as a function of the program temperature (proportional to the measurement time). Characteristic temperature values of DSC signals (onset / endset temperature, peak maximum) are determined from the sample temperature, which may deviate from the program temperature. Signals with positive area correspond to endothermic events.

| | |
|---|---|
| Apparatus: | Mettler-Toledo DSC 822E coupled with a Mettler-Toledo Gas-Flow-Controller TS0800GC1 (Mettler-Toledo GmbH, Gießen, Germany) |
| Aluminium crucible: | 40 µL (with perforated lid) |
| Temperature range: | 30°C to 350°C |
| Heating rate: | 10°C/ min |
| Nitrogen flow: | 50 mL / min |
| Software: | STARe Version 12.00a |
| Interpretation: | Endothermic mode |

### Thermogravimetry (TGA)

The samples were placed in open aluminum crucibles (40 µL). The measured weight curve is displayed as a function of the program temperature.

| | |
|---|---|
| Apparatus: | Mettler-Toledo TGA/DSC1 (Mettler-Toledo GmbH, Gießen, Germany) |
| Aluminium crucible: | 40 µL (open) |
| Temperature range: | 25°C to 300°C |
| Heating rate: | 10°C / min |
| Nitrogen flow: | 50 mL / min |
| Software: | STARe Version 11.00 |

### High Pressure Homogenization

High Pressure Homogenization was performed using a Nano DeBEE 45 laboratory homogenizer (BEE International, 46 Eastman Street, South Easton, MA 02375, USA).

### Preparation of OCA:CD complexes

### A) Preparation of Amorphous OCA:CD Complexes

### a) Preparation by Dissolution / Lyophilization

### Example 1:

### Preparation of OCA:β-CD complex:

OCA (2.59 g; 6.2 mmol) and β-CD (7.0 g; water content by TGA = 13.0%) were magnetically stirred in water (445 mL) at RT for 48h. The obtained suspension was filtered and the clear solution was lyophilized. The yield was 5.6 g (64% based on calculated dry weight; water content according to TGA = 5.3%). The product is amorphous and has an API content of 21.8%.

### Example 2:

### Preparation of OCA:HP-β-CD complex:

OCA (7.50 g; 17.8 mmol) was added to a solution of HP-β-CD (30.0 g; water content by TGA = 5.2%) in water (150 mL). The suspension was stirred at RT for 3 days. The suspension was filtered and the filtrate lyophilized. The yield was 35.5 g (94% based on calculated dry weight; water content according to TGA = 4.1%). The product is amorphous and has an API content of 20.0%.

### Example 3:

### Preparation of RM-β-CD complex:

OCA (1.61 g; 3.8 mmol) was added to a solution of RM-β-CD (5.0 g; water content by TGA = 2.8%) in water (25 mL). The suspension was stirred at RT for 24 hours. The suspension was filtered and the filtrate lyophilized. The yield was 5.8 g (88% based on calculated dry weight; water content according to TGA = 2.6%). The product is amorphous and has an API content of 24.0%.

### Example 4:

### Preparation of SB-β-CD complex:

OCA (0.99 g; 2.3 mmol) was added to a solution of SB-β-CD (5.0 g; water content by TGA = 6.2%) in water (76 mL). The suspension was stirred at RT for 17 hours. The suspension was filtered and the filtrate lyophilized. The yield was 5.7 g (95% based on calculated dry weight; water content according to TGA = 9.2%). The product is amorphous and has an API content of 13.9%.

### b) Preparation by Nano DeBEE high pressure homogenization / Lyophilization

### Example 5a:

### Preparation of OCA:β-CD complex:

25 mL of water are placed in the high pressure homogenizer (Nano DeBEE). OCA (0.352 g; 0.8 mmol) and β-CD (2.0 g; water content by TGA = 13.0%) were added in portions and the obtained suspension was circulated in the Nano DeBEE for 30 minutes at 10 000 psi. Thereafter, the suspension was collected and lyophilized. The yield was 1.77 g (82% based on calculated dry weight; water content according to TGA = 5.4%). The product is amorphous and has an API content of 13.7%.

### Example 5b:

### Preparation of OCA:β-CD complex:

25 mL of water are placed in the high pressure homogenizer (Nano DeBEE). OCA (1.07 g; 2.5 mmol) and β-CD (4.0 g; water content by TGA = 13.0%) were added in portions and the obtained suspension was circulated in the Nano DeBEE for 30 minutes at 10 000 psi. Thereafter, the suspension was collected and lyophilized. The yield was 3.26 g (68% based on calculated dry weight; water content according to TGA = 4.8%). The product is amorphous and has an API content of 18.5%.

### B) Preparation of Crystalline OCA:β-CD Complexes

Amorphous OCA was used as starting material.

### Example 6:

### Preparation of form H33 of OCA: β-CD complex

OCA (3.93 g; 9.3 mmol) and β-CD (12.0 g; 9.3 mmol; water content by TGA = 11.6%) were magnetically stirred in deionized water (50 mL) at RT for 1h. Additional water (610 mL) was added and the suspension was stirred for 2h at RT. A small amount of undissolved solid was removed by filtration through a folded filter. The obtained clear solution was concentrated slowly by open storage in a crystallization dish at RT. After two days the volume had decreased to 120 mL. The crystallized solid was isolated by vacuum filtration and dried for 5 days at RT (23°C) in a desiccator over an aqueous solution of magnesium chloride (MgCl₂) containing excess solid magnesium chloride hexahydrate (MgCl₂ · 6 H₂O) (this corresponds to a relative humidity of approx. 33%). The yield was 7.53 g (46% based on calculated dry weight; water content according to TGA = 11.3%).

### Example 7:

### Preparation of form H33 of obeticholic acid β-cyclodextrin complex

OCA (322 mg; 0.8 mmol) and β-CD (1.0 g; 0.8 mmol; water content by TGA = 13%) were magnetically stirred in deionized water (2 mL) at RT for 1h. Additional water (53 mL) was added and the suspension was stirred for 2h at RT. A small amount of undissolved solid was removed by filtration through a folded filter. Seed crystals (50 mg; form H33 prepared as in Example 1) were added and the solution was stored in a closed flask at 4°C for 9 days. The crystallized solid was isolated by vacuum filtration and dried for 2 days at RT (23°C) in a desiccator over a thick slurry of NaBr and water (approx. 58% relative humidity), then 1 day in a desiccator over an aqueous solution of MgCl₂ containing excess solid MgCl₂ · 6 H₂O (approx. 33% relative humidity). The yield was 530 mg (39% based on calculated dry weight; water content according to TGA = 11.3%)

### Characterization of OCA:CD Complexes

### Experiment 1a: Solid-State Characterization of Amorphous OCA:CD Complexes (Examples 1-5)

| **Example** | **Yield** | **Drug load** | **Water** | **XRD** | **Figure** |
|---|---|---|---|---|---|
| Ex. 1 | 5.6 g (64%) | 21.8% | 5.3% | amorphous | Figure 1 |
| Ex. 2 | 35.5 g (94%) | 20.0% | 4.1% | amorphous | Figure 2 |
| Ex. 3 | 5.8 g (88%) | 24.0% | 2.6% | amorphous | Figure 4 |
| Ex. 4 | 5.7 g (95%) | 13.9% | 9.2% | amorphous | Figure 3 |
| Ex. 5a | 1.7 g (82%) | 13.7% | 5.4% | amorphous | Figure 5a |
| Ex. 5b | 3.3 g (68%) | 18.5% | 4.8% | amorphous | Figure 5b |

### Experiment 1b: Solid-State Characterization of Crystalline OCA:CD Complexes (Examples 6 and 7)

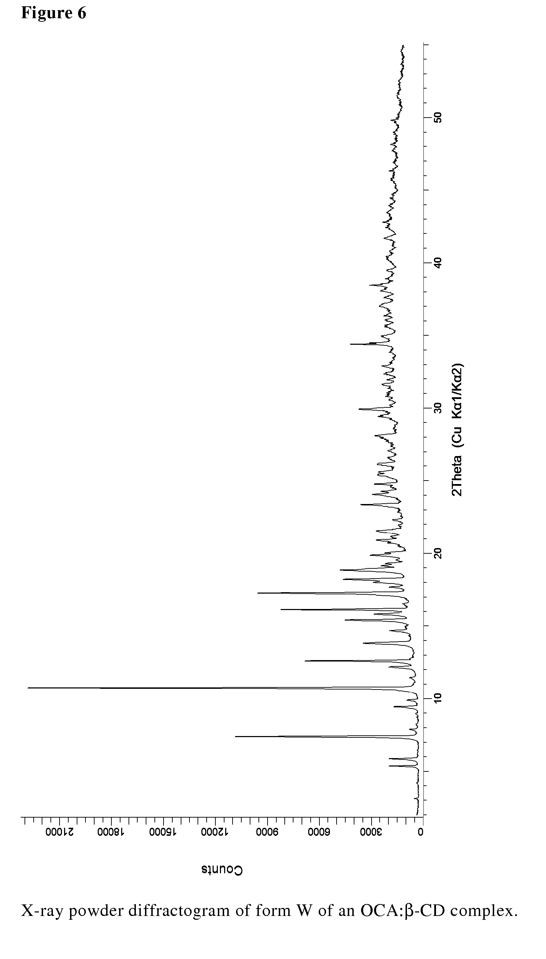

The crystalline OCA: β-CD complex was prepared as in Examples 6 or 7. The crystal lattice of the complex contains water. The amount of crystal water in the solid depends on the relative humidity of the surrounding atmosphere. The crystal structure, as monitored by **X-ray** powder diffraction (XRPD), varies with its water content. A powder diffractogram, which was measured directly after isolation of the still wet solid, is shown in Figure 6. The solid state form defined by this diffractogram is denominated as form W.

XRPD signals for the form W were observed at the following positions: 3.1 (1); 4.2 (0.4); 5.4 (8); 5.9 (7); 7.4 (46); 7.9 (2); 8.2 (1); 9 (1); 9.4 (6); 9.9 (3); 10.7 (100); 11.4 (1); 12.2 (7); 12.6 (29); 13.8 (13); 14.7 (6); 14.9 (1); 15.4 (17); 15.8 (9); 16.1 (34); 16.5 (1); 17.3 (39); 17.7 (5); 18 ( 9); 18.2 (17); 18.8 (17); 19.1(7); 19.3 (5); 19.6 (2); 19.9 (9); 20 (5); ± 0.2 degrees 2-theta. The respective percentage signal intensities were given in brackets after each position.

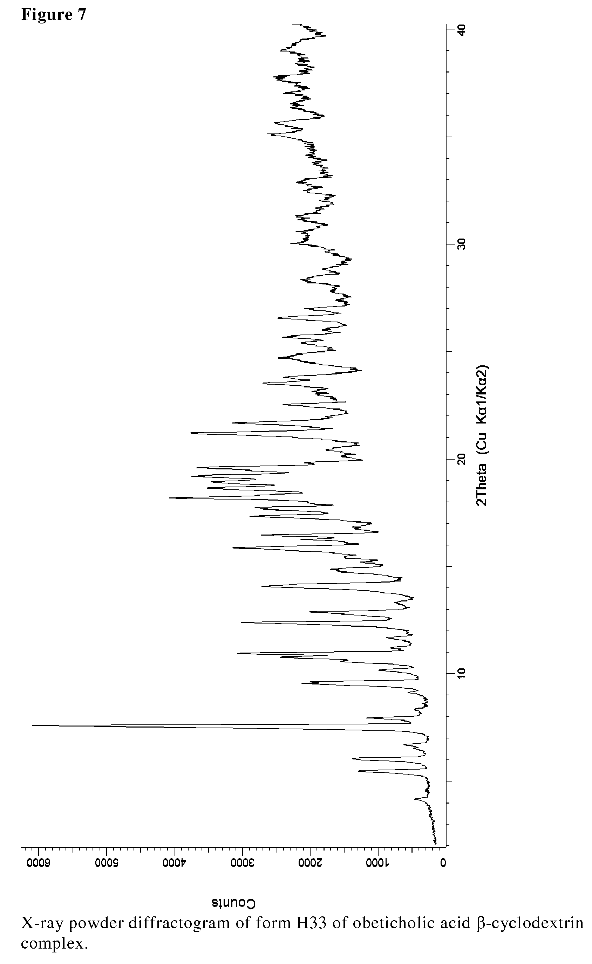

A convenient method of preparing the complex in a reproducible solid state form consists of equilibrating the wet material with an atmosphere of approx. 33% relative humidity (RH). Practically this is accomplished by storing the material at 23°C in a desiccator over an aqueous solution of MgCl₂ containing excess solid MgCL · 6 H₂O (cf. L. Greenspan, Journal of Research of the National Bureau of Standards 1977, 81A, 89; and A. Wexler, S. Hasegawa, Journal of Research of the National Bureau of Standards 1954, 53, 19). The diffractogram of the solid state form produced by this method is shown in Figure 7. This form is denominated as form H33.

XRPD signals for the form H33 were observed at the following positions: 4.2 (4); 5.5 (19); 6.0 (19); 6.6 (4); 6.7 (6); 7.6 (100); 7.9 (15), 8.3 (3); 9.1 (4); 9.6 (28); 10.2 (11); 10.6 (20); 10.8 (36); 10.9 (47); 11.2 (7); 11.7 (7); 12.4 (44); 12.9 (27); 13.3 (4); 14.1 (38); 14.8 (16); 15.2 (9); 15.4 (13); 15.9 (42); 16.2 (23); 16.4 (30); 16.8 (6); 17.3 (34); 17.7 (30); 18 (18); 18.2 (54); 18.6 (43); 18.9 (41); 19.2 (45); 19.5 (32); 19.6 (45); 19.8 (15); 20.1 (6); ± 0.2 degrees 2-theta. The respective percentage signal intensities were given in brackets after each position.

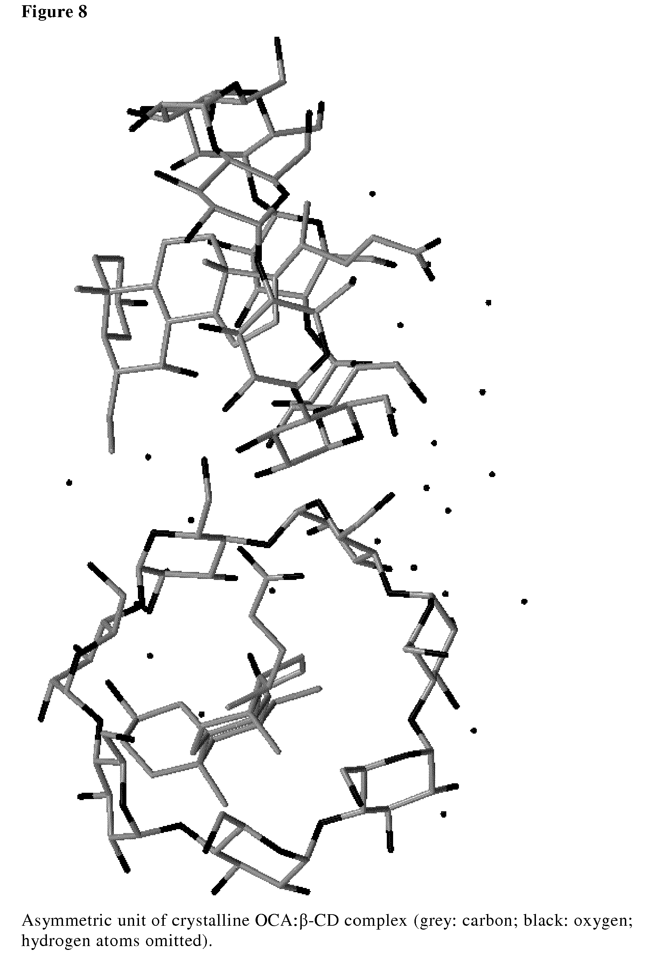

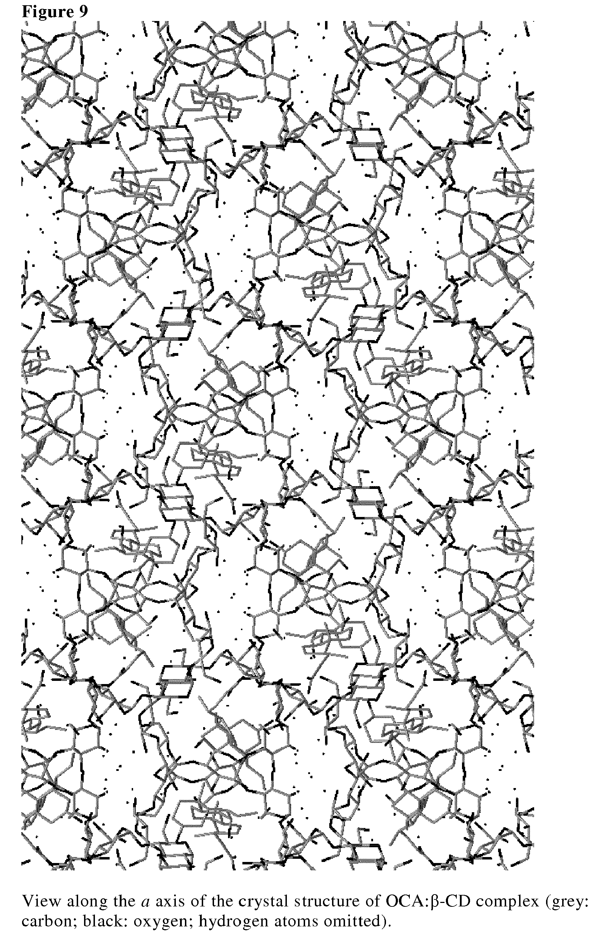

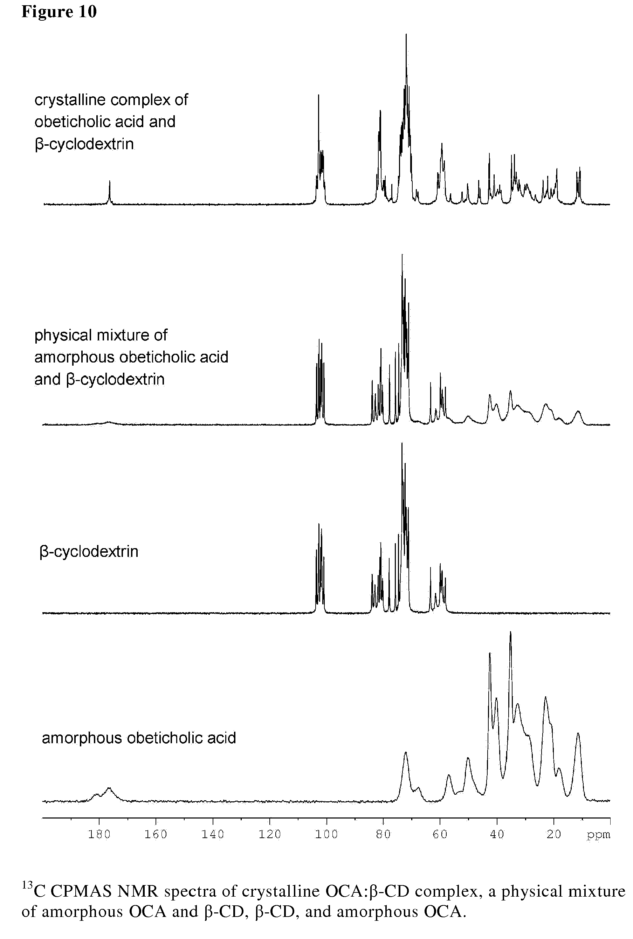

The obeticholic acid content determined by HPLC ranges from 23.5 to 23.9%, corresponding to a water content of 12 - 13% (calculated for a 1:1 ratio of OCA : β-CD). The crystal structure was determined by single crystal X-ray diffraction and indicates a stoichiometry of [(OCA)₁(β-CD)₁(H₂O)₁₃], corresponding to 13.1% of water, although the exact number of water molecules could not be determined with certainty due to the complexity of the structure. Figure 8 shows the asymmetric unit of the crystal structure (orthorhombic, space group *P*2₁2₁2₁), which contains two complexes and 26 water molecules. The OCA molecules are inserted in the CD rings (inclusion complex). Figure 9 shows a view along the *a* axis of the structure. It is obvious that the structure contains channels between the complexes, which are occupied with water molecules. This explains the varying water content of the solid depending on the relative humidity of the atmosphere, because the channels allow exchange of water molecules between solid and gaseous phase. Figure 10 gives a comparison of ¹³C solid state NMR spectra (CPMAS) of the crystalline complex, a physical mixture of amorphous obeticholic acid and β-CD, and the pure components. The effects of complexation on the line widths and peak positions of obeticholic acid are clearly recognizable.

No changes were observed when form H33 is stored for 2 days at 23°C and approx. 23% RH (desiccator with a thick slurry of potassium acetate and water). When Form H33 is handled in a laboratory atmosphere with RH below 23% (but above 11%, vide infra), a conversion of the solid state form into a new form (form HLT23, i.e. humidity of less than 23%) is observed.

XRPD signals for the form HLT23 were observed at the following positions: 3.2 (1); 4.8 (3); 5.5 (27); 6.4 (38); 6.6 (11); 7.8 (65); 8.0 (8); 8.8 (7); 9.2 (4); 9.6 (34); 9.7 (11); 10.6 (15); 11.0 (75); 12.1(21); 12.5 (57); 13.2 (16); 13.4 (9); 13.8 (15); 14.5 (19); 14.8 (46); 15.5. (8); 16.7 (100); 17.1 (13); 17.6 (50); 18.0 (53); 18.2 (50); 18.4 (54); 19.2 (52); 19.4 (45); 19.8 (36); 20.0 (34); ± 0.2 degrees 2-theta. The respective percentage signal intensities were given in brackets after each position.

Thermogravimetric measurement indicates a weight loss of approx. 8% up to 120°C. The transformation of form H33 into form HLT23 is reversible: when a sample of form HLT23 is stored for 1.5 days at 23°C and 33%RH it is converted back into form H33.

When form H33 was stored at 23°C and 11% RH (desiccator with a thick slurry of lithium chloride and water) it is converted into a solid state form of low crystallinity, which is denominated as form H11. Thermogravimetric measurement indicates a weight loss of approx. 5 - 6% up to 120°C.

XRPD signals for the form H11 were observed at the following positions: 5.4; 6.9; 9.2; 10.0; 10.9; 12.4; 15.7; 16.5; 18.0; ± 0.2 degrees 2-theta.

Changes in the diffractogram of the crystalline OCA:β-CD complex occur not only at the specific values of relative humidity which are described here. Although the diffractograms of forms W, H33, HLT23, and H11 represent distinguished solid state forms of the complex, there exist more variations in the diffractogram in dependence of relative humidity.

### Experiment 1c: Single-Crystal Diffraction of Crystalline OCA:CD Complexes (Examples 6 and 7)

A crystal of obeticholic acid β-cyclodextrin complex (originally form H33, as described above, water content may have slightly changed during storage) of dimensions 0.05 x 0.07 x 0.21 mm was measured according to the above-described methods.

The asymmetric unit comprises two complexes, which have both 1:1 stoichiometry. The crystal lattice contains water. The number of water molecules per complex was determined as 13. Due to possible fractional occupation of water sites this number is not completely certain. Only hydrogen atoms which were found in the difference map were included in the structure model (refined initially with soft restraints, then according to a riding model). Hence their number is lower then expected. The data obtained are shown in the Table below:

| Empirical formula | C₁₃₆H₂₁₁O₁₀₄ |
|---|---|
| Formula weight | 3510.14 |
| Temperature | 180 K |
| Wavelength | 1.54178 Å |
| Crystal system | orthorhombic |
| Space group | *P*2₁2₁2₁ |
| Unit cell dimensions | |
| *a*[Å] | 19.3103(4) |
| *b*[Å] | 28.9771(7) |
| *c*[Å] | 30.5639(7) |
| Volume [Å³] | 17102.2(4) |
| Z | 4 |
| Density (calculated) [g·cm⁻³] | 1.363 |
| Absorption coefficient [mm⁻¹] | 1.029 |
| *F*(000) | 7436 |
| Crystal size [mm] | 0.05 x 0.07 x 0.21 |
| Theta range for data collection | 2.10-66.70 |
| Index ranges | -22≤*h*≤22, -34≤*k≤*33, -36≤*l*≤36 |
| Reflections collected | 114804 |
| Independent reflections [*R*(int)] | 29868 (0.063) |
| Completeness to Theta = 64.03° | 99.2% |
| Absorption correction | Semi-empirical from equivalents |
| Max. and min. transmission | 0.95 and 0.23 |
| Refinement method | Full-matrix least-squares on *F*² |
| Data / parameters | 29868 / 2137 |
| Goodness-of-fit on *F²* | 0.8445 |
| Final *R* indices [*I*>2σ(*I*)] | *R₁* = 0.0833, w*R*₂ = 0.2139 |
| Flack parameter | 0.11(15) |
| Final *R* indices [all data] | *R₁* = 0.0942, w*R*₂ = 0.2227 |
| Largest diff. peak and hole [e·Å⁻³] | 1.13 and -0.63 |

### Experiment 2: Determination of solubility of OCA in concentrated CD solutions

Variable amounts of solid OCA (left column of the Tables given below) were stirred in aqueous CD-solutions (14% (w/w) α-CD, 1.8% β-CD, 20% γ-CD, 20% HP-β-CD, 30% SB-β-CD, 30% RM-β-CD, and 40% HP-γ-CD) for 60 h. Aliquots were filtered through 0.2 µm disposable PTFE filters and the filtrates were analyzed by HPLC/UV. The concentration of OCA was determined based on a calibration curve which was established by twofold analysis of five calibration standards with 0.01 mg/mL to 1.0 mg/mL OCA. The weighed amounts of OCA in test solutions and the determined amount of dissolved OCA are summarized in the Table below.

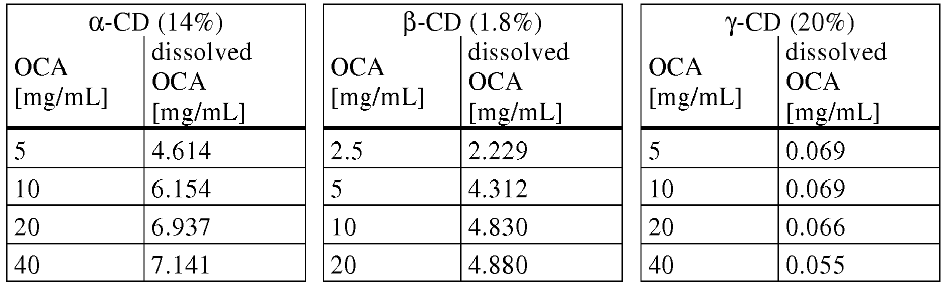

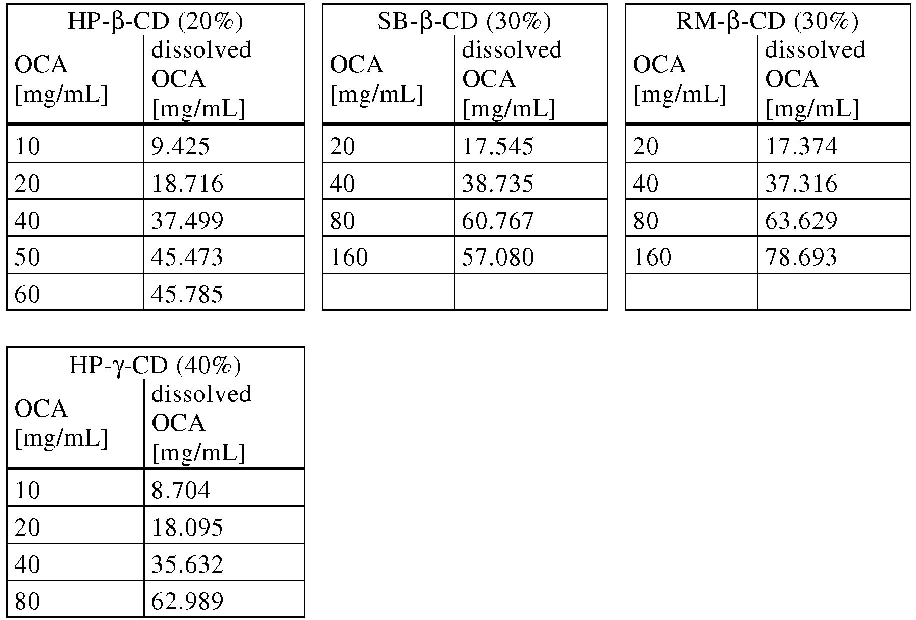

### Experiment 3: Determination of solubility isotherms of OCA in aqueous CD solutions.

Experiments were performed to determine solubility isotherms which provide information regarding the characteristics of the OCA:CD complexes. The solubility isotherms are generated by determination of the dissolved OCA [mol/L] as a function of CD concentration [mol/L].

Aqueous cyclodextrin (CD) stock solutions were prepared in glass flasks by mixing solid CD with distilled water, followed by ultrasonic treatment. Concentrations of stock solutions correspond with the maximum aqueous solubility of the used CD at RT. To determine the solubility of OCA as a function of CD concentration, OCA was weighed into a vial (cf. the Table given below), the CD solution (2 mL) was added and the resulting suspension was stirred on a magnetic stirrer (500 rpm) for 60 h at RT. Thereafter, the suspension was filtered through a disposable filter (0.2 µm PTFE filter) and the clear filtrate was analyzed by HPLC/UV.

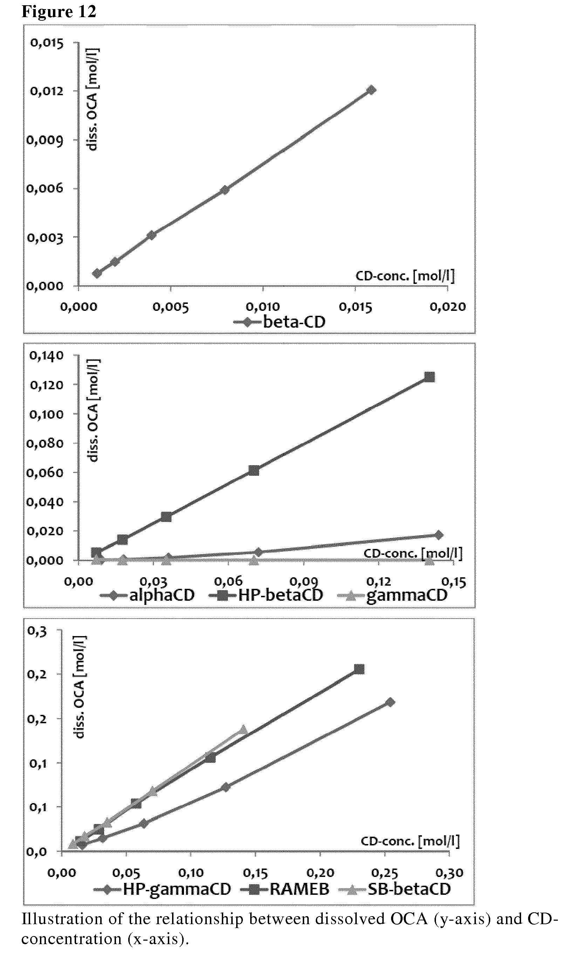

The relationship between CD concentration and dissolved OCA is graphically illustrated in Figure 12, the determined slopes of isotherms are given in the Table below. The Table contains also the amount of OCA used for determination (fourth column) and the examined range of CD concentrations (third column).

| **Cyclodextrin** | | | **OCA Weight (mg/mL)** | **Slope of isotherm** |
|---|---|---|---|---|
| **Type** | **Saturation solubility (mol/L)** | **Concentration range (%)** | | |
| α-CD | 0.149 | 0.875 - 14 | 20 | 0.127 |
| β-CD | 0.016 | 0.113 - 1.8 | 10 | 0.759 |
| HP-β-CD | >0.421 | 1 - 20 | 60 | 0.900 |
| RM-β-CD | >0.384 | 1.88 - 30 | 100 | 0.898 |
| SB-β-CD | >0.235 | 1.88 - 30 | 70 | 0.983 |
| γ-CD | 0.179 | 1 - 20 | 1 | N/A |
| HP-γ-CD | >0.381 | 2.5 - 40 | 80 | 0.682 |

### Experiment 4: Physical-Chemical Characterization of crystalline OCA:β-CD complex (1:1)

### Chemical Purity of OCA

The chemical purity of the obeticholic acid contained in the complex was found by HPLC-UV/MS to be identical with the starting material (amorphous obeticholic acid), i.e. close to 100%.

### Solubility of OCA:CD complexes in physiologically relevant media

Solubilities of OCA:CD complexes were determined by stirring an amount of complex which corresponds to 20 mg of obeticholic acid (calculated from the known drug loads) in 2 mL of solvent for 15 min and 60 min at 37°C according to the method described above under "Analytical Methods". Solubility values are given in mg/ml.

| | **Water (pH 7)** | | **0.01 N HCl (pH 2.2)** | | **FassiF* (pH 6.5)** | | **50 mM KH₂PO₄ (pH 6.5)** | |
|---|---|---|---|---|---|---|---|---|
| | 15 min | 60 min | 15 min | 60 min | 15 min | 60 min | 15 min | 60 min |

| **Crystalline complex** | | | | | | | | |
|---|---|---|---|---|---|---|---|---|
| Form H33 | 11.47 | 12.43 | 11.62 | 12.07 | 14.90 | 14.64 | 16.22 | 17.10 |
| Form HTL23 | 11.03 | 12.01 | 10.65 | 11.74 | 15.16 | 16.09 | 17.23 | 17.25 |
| Form H11 | n.d.** | 11.54 | n.d. | 11.87 | n.d. | 15.18 | n.d. | 17.20 |

| **Amorphous complex** | | | | | | | | |
|---|---|---|---|---|---|---|---|---|
| Ex. 1 | 12.08 | 11.69 | 12.62 | 11.77 | 16.12 | 115.21 | 18.04 | 17.82 |
| Ex. 2 | 18.76 | 18.51 | 17.18 | 17.42 | 17.44 | 17.42 | 17.58 | 18.25 |
| Ex. 3 | 18.43 | 20.03 | 18.29 | 20.80 | 18.46 | 19.64 | 18.73 | 17.87 |
| Ex. 4 | 19.08 | 21.10 | 19.64 | 20.30 | 18.99 | 21.16 | 19.05 | 20.73 |
| Ex. 5b | 18.76 | 18.51 | 17.18 | 17.42 | 17.44 | 17.42 | 17.58 | 18.25 |
| **Comp. Ex. OCA amorph***** | <0.01 | <0.01 | <0.01 | <0.01 | 0.52 | 0.42 | 0.11 | 0.11 |

| | | | | | | | | |
|---|---|---|---|---|---|---|---|---|
| * Fasted State Simulated Intestinal Fluid ** not determined *** water solubility reported in WO 2013/192097 and measured in 0.1 N HCl after equilibration under stirring for one week at 25°C is 17.9 µmol/l (0.0075 mg/ml) | | | | | | | | |

### Hygroscopicity

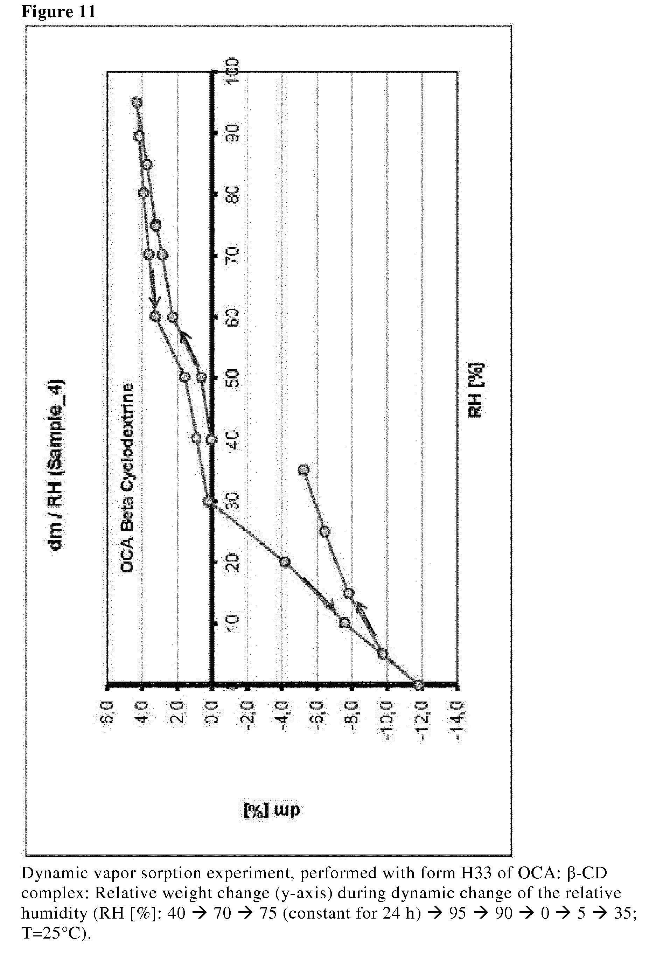

The result of a representative DVS measurement with crystal form H33 of obeticholic acid β-cyclodextrin complex is shown in Figure 11.

Starting from 40% RH the complex takes up to 4.3% of water until 95% RH. Upon lowering of the humidity the complex looses up to 11.8% of the initial weight at 0% RH. The material which remains after the final raising of the humidity to 35% RH contains 5.2% less of water than at the starting point. Powder diffraction reveals that it is largely amorphous.

### Effects of the present invention and industrial applicability

In the above-described experiments, the interaction between OCA and a series of CD-derivatives was investigated. Solubility tests in saturated CD-solutions suggested an interaction between OCA and various CDs. The solubility isotherms suggested the formation of complexes with 1:1 stoichiometry.

Further, OCA-complexes were exemplary prepared with β-CD, RM-β-CD, SB-β-CD and HP-β-CD. To this end, the components were dissolved in water, followed by freezing and freeze-drying. XRPD analysis of the products demonstrated that all samples, which were prepared with this procedure, were amorphous.

In parallel, an exemplary solution of OCA and β-CD was kept in a crystallization dish and upon slow evaporation of water a unique crystalline product was obtained. ¹H-NMR analysis of the crystalline product demonstrated the presence of equimolar amounts of OCA and β-CD. The solid-state ¹³C-CPMAS spectrum is characterized by 91 well-resolved signals, compared to 26 and 42 carbons of OCA and β-CD, respectively. The shape of the signals suggest that both components are present in crystalline form, the chemical shifts of the ¹³C-signals relative to those of the individual components as well as of the physical mixture support the interpretation of a defined new crystalline complex. The presence of 91 signals shows that the unit cell contains more than one molecule of OCA and/or CD. Furthermore, thermogravimetric and XRPD analyses of samples, which were stored at different environmental humidity, demonstrated a certain but not unlimited flexibility regarding water content and crystalline nature. Four discrete forms, denominated as form W, H33, HLT23 and H11 have been defined and the crystal structure of form H33 was analyzed by single crystal X-ray diffraction. Results indicated a stoichiometry of [(OCA)₁(β-CD)₂(H₂O)₁₃], corresponding to 13.1% of water.

Solubility experiments in water, 0.01 N HCl, FaSSIF, and phosphate buffer, pH 6.5 demonstrated that the OCA:β-CD complexes exhibit a pH-independent solubility of OCA. The highly similar solubility of the OCA:βCD-complex in FaSSIF and phosphate buffer, both pH 6.5, was surprising, because FaSSIF contains taurodeoxycholic acid, another bile acid derivative, which could potentially interfere with the OCA-βCD complex and modify the solubility of OCA.

The solubility characteristics of crystalline and amorphous complexes are highly similar and basically, both products can be used as an OCA formulation with pH-independent solubility.

In summary, the prior art problems of intervariable and pH-dependent solubility of obeticholic acid could be overcome in the present invention by the provision of a complex compound comprising obeticholic acid and cyclodextrin. Both amorphous and crystalline complexes could be provided, which both demonstrate a high and pH-independent solubility in various aqueous media. Thus, the complexes may beneficially be used for the manufacture of pharmaceutical formulations, which constantly release the active compound obeticholic acid to the subject. Such formulations may thus be highly suitable for use in the treatment of hepatic or biliary diseases and conditions.

## Claims

1. A complex compound comprising:
(a) obeticholic acid and
(b) cyclodextrin.

2. The complex compound according to claim 1, which is an inclusion complex.

3. The complex compound according to claim 1 or 2, wherein the molar ratio of obeticholic acid to cyclodextrin is about 1:1.

4. The complex compound according to any one of the previous claims, wherein cyclodextrin is selected from β-cyclodextrin, (2-hydroxypropyl)-β-cyclodextrin, (2-hydroxypropyl)-γ-cyclodextrin, random methyl-β-cyclodextrin and sodium sulfobutylether-β-cyclodextrin.

5. The complex compound according to any one of claims 1 to 4, which is present in amorphous form.

6. The complex compound according to any one of claims 1 to 4, which is present in crystalline form.

7. The complex according to claim 6, in which the crystalline form is **characterized by** an **X-ray** diffraction pattern including characteristic peaks at about 5.5, 7.6; 10.9; 12.4; and 18.2 degrees 2-theta ± 0.2 degrees 2-theta.

8. A crystalline complex compound comprising:
(a) obeticholic acid and
(b) cyclodextrin,
wherein the complex is prepared by the steps of:
(i) concentrating or cooling and optionally adding seed crystals to a saturated solution of obeticholic acid and cyclodextrin in an aqueous solvent,
(ii) isolating crystals of the complex compound from the suspension obtained in step (i), and
(iii) drying of the crystals in a desiccator over an aqueous solution of magnesium chloride containing excess solid magnesium chloride hexahydrate.

9. The crystalline complex compound of claim 8, which is is **characterized by** an **X-ray** diffraction pattern including characteristic peaks at about 5.5, 7.6; 10.9; 12.4; and 18.2 degrees 2-theta ± 0.2 degrees 2-theta.

10. An amorphous complex compound comprising:
(a) obeticholic acid and
(b) β-cyclodextrin,
wherein the complex is prepared by the steps of:
(i) suspending obeticholic acid and β-cyclodextrin in an aqueous solvent,
(ii) circulating the suspension in a high pressure homogenizer and
(iii) lyophilisation of the resulting suspension.

11. A pharmaceutical formulation, comprising the complex compound according to any one of claims 1 to 10, and optionally one or more pharmaceutical excipient(s).

12. The pharmaceutical formulation according to claim 11, which is a tablet or capsule.

13. The complex compound according to any one of claims 1 to 10 for use in the medical treatment of a farnesoid X receptor (FXR) mediated disease or condition.

14. The complex compound for use according to claim 13, in which the FXR mediated disease or condition is a hepatic or biliary disease or condition.

15. The complex compound for use according to claim 13 or 14, wherein the disease or condition is selected from biliary atresia, cholestatic liver disease, chronic liver disease, nonalcoholic steatohepatitis (NASH), hepatitis C infection, alcoholic liver disease, primary biliary cirrhosis (PBC), liver damage due to progressive fibrosis, liver fibrosis, and cardiovascular diseases including atherosclerosis, arteriosclerosis, hypercholesteremia, and hyperlipidemia.
